(19) Europäisches Patentamt
European Patent Office
Office européen des brevets

(11) **EP 2 681 181 B1**

(12) **EUROPÄISCHE PATENTSCHRIFT**

(45) Veröffentlichungstag und Bekanntmachung des Hinweises auf die Patenterteilung:
**21.01.2015 Patentblatt 2015/04**

(51) Int Cl.:
***C07C 45/74*** *(2006.01)*     ***C07C 47/21*** *(2006.01)*

(21) Anmeldenummer: **12712216.6**

(22) Anmeldetag: **09.02.2012**

(86) Internationale Anmeldenummer:
**PCT/EP2012/000523**

(87) Internationale Veröffentlichungsnummer:
**WO 2012/116775 (07.09.2012 Gazette 2012/36)**

(54) **VERFAHREN ZUR DURCHFÜHRUNG VON MEHRPHASIGEN ALDOLKONDENSATIONSREAKTIONEN ZU GEMISCHTEN , -UNGESÄTTIGTEN ALDEHYDEN**

METHOD FOR CARRYING OUT MULTIPHASE ALDOL CONDENSATION REACTIONS TO OBTAIN MIXED ALPHA,BETA-UNSATURATED ALDEHYDES

PROCÉDÉ POUR LA MISE EN OEUVRE DE RÉACTIONS DE CONDENSATION D'ALDOLS MULTIPHASIQUES EN ALDÉHYDES ALPHA,BETA-INSATURÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.03.2011 DE 102011012846**

(43) Veröffentlichungstag der Anmeldung:
**08.01.2014 Patentblatt 2014/02**

(73) Patentinhaber: OXEA GmbH
**46147 Oberhausen (DE)**

(72) Erfinder:
- **BERMANN, Dirk**
  **45475 Mülheim (DE)**
- **FREY, Guido D**
  **64560 Riedstadt (DE)**
- **NOWOTNY, Norman**
  **45276 Essen (DE)**
- **SCHALAPSKI, Kurt**
  **46147 Oberhausen (DE)**
- **STRUTZ, Heinz**
  **47445 Moers (DE)**

(56) Entgegenhaltungen:
**EP-A2- 1 106 596    DE-A1-102009 001 594**

**Beschreibung**

Die vorliegende Erfindung betrifft ein Verfahren zur Durchführung von mehrphasigen Aldolkondensationsreaktionen zu gemischten $\alpha,\beta$-ungesättigten Aldehyden in einem Rohrreaktor unter laminaren Bedingungen.

Die Aldolkondensationsreaktion von gesättigten Aldehyden unter Wasserabspaltung zu dem entsprechenden ungesättigten Dimer oder $\alpha,\beta$-ungesättigten Aldehyd ist eine in der organischen Chemie geläufige Reaktion. Die Umsetzung kann mit nur einer bestimmten Aldehydverbindung in einer Selbstkondensationsreaktion erfolgen oder aber auch zwischen Aldehyden mit einer unterschiedlichen Kohlenstoffzahl in einer gemischten Aldolkondensationsreaktion. Damit es nach der Addition zweier Aldehydmoleküle zum $\beta$-Hydroxyaldehyd zu der intramolekularen Wasserabspaltung und damit zur Ausbildung der $\alpha,\beta$-ungesättigten Bindung kommen kann, muss bei der Aldolkondensationsreaktion mindestens ein Aldehyd zugegen sein, der an dem zur Carbonylgruppe $\alpha$-ständigen Kohlenstoffatom zwei Wasserstoffatome trägt.

Die gezielte Unterdrückung der Wasserabspaltung aus der $\beta$-Hydroxyalde-hydzwischenstufe ist eher auf Sonderfälle beschränkt. Gemäß der WO 95/07254 A1 wird die Aldoladdition von n-Butyraldehyd mit einer wässrigen Natronlauge in Gegenwart von Polyethylenglykol bei niedrigen Temperaturen durchgeführt. Nach Neutralisation des basischen Katalysators und Abtrennung der wässrigen Phase wird die organische Phase, die 2-Ethyl-3-hydroxyhexanal enthält, hydriert. Als Zielprodukt wird 2-Ethyl-1,3-hexandiol angestrebt und die Bildung von 2-Ethylhexanol soll möglichst zurückgedrängt werden.

Von größerer technischer Bedeutung ist jedoch die Herstellung von 2-Ethylhexanol durch die Aldolkondensationsreaktion. Der Alkohol wird ausgehend von n-Butyraldehyd in Gegenwart eines alkalischen Katalysators, üblicherweise einer wässrigen Natronlauge, bei erhöhter Temperatur über die unter Wasserabspaltung gebildete Zwischenstufe 2-Ethylhexenal hergestellt. Der $\alpha,\beta$-ungesättigte Aldehyd wird anschließend zu 2-Ethylhexanol hydriert, üblicherweise in der Gasphase in einer ersten Stufe mit einer nachfolgenden Flüssigphasenhydrierung in der zweiten Stufe (EP 0 420 035 A1). Durch das freigesetzte Reaktionswasser verdünnt sich die wässrige Natronlauge, die ebenfalls mit organischen Verunreinigungen, wie Butyraten, belastet wird. Daher muss stets ein Teil der verdünnten und belasteten wässrigen Natronlauge ausgeschleust und durch Frischlauge ersetzt werden. Die Aldolkondensation von n-Butyraldehyd in Gegenwart einer wässrigen Natronlauge kann beispielsweise in einem Rührkessel, wie in US 3,763,247 oder DE 927 626 beschrieben, oder in einer gepackten Säule durchgeführt werden, die im Gegenstrom betrieben wird [G. Dümbgen, D. Neubauer, Chemie -Ing.-Tech. 41, 974 (1969)]. Die Reaktion in einem Rührkessel ist jedoch nachteilig, da bei dieser Art der Reaktionsauslegung die Wärmeabfuhr problematisch sein kann und der Betrieb mechanisch bewegter Teile wartungsintensiv und reparaturanfällig ist.

Es ist auch bekannt, n-Butyraldehyd und wässrige Natronlauge in einer Mischpumpe intensiv zu vermischen und das heterogene Gemisch nach Verlassen der Mischpumpe im turbulenten Zustand zu belassen. Nach US 2,468,710 wird das Gemisch durch eine Mischstrecke geführt und anschließend zur Vervollständigung der Reaktion in ein Reaktionsgefäß geleitet, aus dem am Kopf das gewünschte Aldolkondensationsprodukt abgeführt wird und aus dem über einen Sumpfabzug ein Flüssigkeitsstrom wieder zurückgeführt wird. Gemäß GB 761,203 wird das heterogene Gemisch turbulent mittels der Mischpumpe durch ein Strömungsrohr geführt und anschließend in einen Phasentrenner gegeben. Die leichtere organische Phase wird abgeführt während die wässrige Natronlauge teilweise ausgeschleust und teilweise wieder in den Aldolkondensationsprozess zurückgeführt wird. Das bekannte Verfahren kann auch auf die gemischte Aldolkondensationsreaktion unterschiedlicher Aldehyde angewandt werden, beispielsweise auf die Umsetzung von Acetaldehyd mit n-Butyraldehyd, die zu einem Gemisch aus $\alpha,\beta$-ungesättigten Aldehyden führt, in dem die Verbindungen aus der Selbst- und Mischaldolisierung vorliegen.

EP 1 106 596 A2 behandelt die Durchführung der Aldolkondensationsreaktion in einem Rohrreaktor. Das bekannte Verfahren ist durch die Angabe eines Belastungsfaktors charakterisiert, der mindestens einzustellen ist, um den turbulenten Betrieb des Rohrreaktors zu gewährleisten. Kennzeichnend ist das hohe Verhältnis der Massenströme von der wässrigen Katalysatorphase zu der Aldehydphase, so dass die wässrige Katalysatorphase die kontinuierliche Phase bildet, in der die Aldehydphase in feinen Tropfen dispergiert vorliegt. Gegebenenfalls kann die wässrige Katalysatorphase noch ein wasserlösliches Lösungsmittel, wie Diethylenglykol, enthalten, um den Stoffaustausch zwischen der wässrigen Katalysatorphase und der organischen Aldehydphase zu erleichtern. Beispielhaft wird die Selbstaldolkondensation von n-Pentanal oder 3-Methylbutanal behandelt sowie die Aldolkondensation einer Aldehydmischung aus n-Pentanal und 2-Methylbutanal.

Die Aldolkondensation von aliphatischen Pentanalen zu $\alpha,\beta$-ungesättigten $C_{10}$-Aldehyden wird ebenfalls in der WO 2010/105892 A1 beschrieben. Auch bei diesem Verfahren wird in einem Rohrreaktor gearbeitet, wobei die organische Phase in der wässrigen Katalysatorphase in Form von Tropfen dispergiert wird. Dabei liegt der durchschnittliche Sauter-Durchmesser der Tropfen zwischen 0,2 und 2 mm. Diese Tropfengröße kann mit einem geringen Energieeinsatz eingestellt werden und gewährleistet gleichzeitig einen hohen Stoffaustausch zwischen der dispergierten organischen Phase und der kontinuierlichen wässrigen Katalysatorphase.

Die bekannten Verfahren zur Durchführung der Aldolkondensation zu $\alpha,\beta$-ungesättigten Aldehyden erfordern

einen hohen Energieeintrag, um ein Strömungsrohr oder um einen Rohrreaktor im turbulenten Zustand zu betreiben. Der Stand der Technik schlägt entweder den Einsatz von Mischpumpen mit einem nachfolgenden Strömungsrohr vor oder empfiehlt bei einem Rohrreaktor ein hohes Verhältnis der Massenströme von der wässrigen Katalysatorlösung zur zugeführten Aldehydphase. Nachteilig bei diesem Verfahren ist jedoch die Verwendung eines sehr großen Massenstroms an wässriger Katalysatorlösung, so dass pro Zeit- und Volumeneinheit nur eine geringe Menge an organischem Produkt durch den Reaktor geführt wird. Wird zusätzlich ein wasserlösliches Lösungsmittel verwendet, erhöht sich der Reinigungsaufwand für das gewünschte Produkt. Auch ist bei Verwendung grenzflächenaktiver Substanzen mit einer erhöhten Belastung des ausgeschleusten Aldolisierungsabwassers mit organischen Verunreinigungen zu rechnen. Der Betrieb von Mischpumpen ist energieintensiv und erhöht den Wartungsaufwand. Ebenfalls erfordert die Aldolkondensationsreaktion höherer Aldehyde, wie $C_5$-Aldehyde und höher, eine vergleichsweise hohe Reaktionstemperatur, um eine möglichst vollständige Wasserabspaltung aus der β-Hydroxyaldehyd-Zwischenstufe zu dem α,β-ungesättigten Aldehyd zu erzielen. Wenn das gewünschte Zielprodukt aus der Mischaldolisierung zweier verschiedener Aldehyde resultieren soll, sind die Reaktionsbedingungen so einzustellen, dass die Selbstkondensation der jeweiligen Einsatzaldehyde möglichst in den Hintergrund gedrängt wird.

Aufgabe der vorliegenden Erfindung ist es daher, ein Verfahren zur Durchführung von mehrphasigen Aldolkondensationsreaktionen zu gemischten α,β-ungesättigten Aldehyden bereitzustellen, das einen geringen technischen Aufwand erfordert, wenig energieintensiv ist und das sich durch eine hohe Raum-Zeit-Ausbeute an gemischten Aldolkondensationsprodukten oder α,β-ungesättigten Aldehyden auszeichnet. Des Weiteren soll das Gefahrenpotential, das mit dem Arbeiten mit niedrigen, ausgesprochen flüchtigen Aldehyden, die eine geringe Zündtemperatur aufweisen, einhergeht, minimiert werden. Ebenfalls sollen die gemischten Aldolkondensationsprodukte mit hohem Umsatz und mit hoher Selektivität erhalten werden und die Bildung von α,β-ungesättigten Aldehyden aus der Selbstkondensation sowie die Bildung von Nebenprodukten, insbesondere die Bildung von hochsiedenden Kondensationsprodukten, zurückgedrängt werden.

Gegenstand der vorliegenden Erfindung ist daher ein kontinuierliches Verfahren zur Durchführung einer mehrphasigen Aldolkondensationsreaktion zu gemischten α,β-ungesättigten Aldehyden in einem Rohrreaktor durch Umsetzung von zwei aliphatischen Aldehyden mit unterschiedlicher Kohlenstoffzahl, die 2 bis 5 Kohlenstoffatome im Molekül enthalten. Das Verfahren ist dadurch gekennzeichnet, dass die aliphatischen Aldehyde in einem Molverhältnis von 1:1 bis 5:1 separat gemischt werden, danach die Aldehydmischung und eine wässrige Lösung einer basisch reagierenden Verbindung über den Boden in einen aufrecht stehenden Rohrreaktor im Gleichstrom eingeleitet werden, wobei die wässrige Lösung der basisch reagierenden Verbindung die kontinuierliche Phase bildet, die unter laminaren Bedingungen durch den Rohrreaktor strömt, und die Aldehydmischung die in der kontinuierlichen Phasen tropfenförmig dispergierte Phase bildet und wobei die Aldehydkondensationsreaktion bei einer Temperatur bis 90°C durchgeführt wird und wobei der Rohrreaktor mit einer Belastung V/Vh mit dem Aldehydgemisch pro Reaktorvolumen und Zeit von 0,1 bis 1,3 betrieben wird.

Unter dem Begriff gemischte α,β-ungesättigte Aldehyde im Sinne der Erfindung werden die Produkte verstanden, die durch die Aldolkondensationsreaktion zweier Aldehyde mit unterschiedlicher Kohlenstoffzahl gebildet werden. Damit die Wasserabspaltung aus dem intermediär gebildeten β-Hydroxyaldehyd erfolgen kann, muss wenigstens eine Aldehydkomponente in der Aldehydmischung zwei Wasserstoffatome an dem zur Carbonylgruppe benachbarten α-Kohlenstoffatom tragen. Die beiden aliphatischen Aldehyde werden zunächst vor dem Eintritt in den Rohrreaktor in einer separaten Einrichtung vorgemischt und dann als Mischung dem Rohrreaktor zugeführt.

Dieses Abmischen kann beispielsweise in einem vorgeschalteten Rührbehälter erfolgen. Je nach Siedepunktslage der verwendeten aliphatischen Aldehyde kann es sich empfehlen, den vorgeschalteten Mischvorgang unter Kühlung oder unter Eigendruck in geschlossenen Gefäßen durchzuführen.

Die organische Phase und die wässrige Phase sind gar nicht oder nur sehr wenig miteinander mischbar und bilden in dem aufrecht stehenden Rohrreaktor ein mehrphasiges Flüssigkeitsgemisch, bei dem die organische Phase die dispergierte Phase bildet und die wässrige Phase, die die basisch reagierende Verbindung oder den Aldolisierungskatalysator enthält, die kontinuierliche Phase ist. Für die kontinuierliche Phase kann das Strömungsverhalten in dem Rohrreaktor nach dem Bodenbereich, in dem die Stoffströme in den Rohrreaktor eintreten, als laminares Strömungsverhalten beschrieben werden.

Um den laminaren Strömungszustand der kontinuierlichen oder wässrigen, katalysatorhaltigen Phase im Rohrreaktor sicherzustellen, sind der Massenstrom, die Stoffwerte Dichte und dynamische Viskosität der kontinuierlichen Phase und der hydraulische Innendurchmesser des Rohrreaktors so in Beziehung zu setzen, dass die charakteristische Reynoldszahl, die den Übergang von dem laminaren Zustand in den turbulenten Zustand anzeigt, nicht überschritten wird. Der formelmäßige Zusammenhang zur Berechnung der Reynolds-Zahl gemäß Gleichung 1:

$$Re = w\,\rho\,d\,/\,\eta \qquad\qquad (1)$$

mit w = Massenstrom [kg/h] der kontinuierlichen Phase, p = Dichte [kg/m³] der kontinuierlichen Phase, d = hydraulischer Innendurchmesser des Rohrreaktors [m] und $\eta$ = dynamische Viskosität [Pa*s] der kontinuierlichen Phase ist allgemein bekannt (VDI Wärmeatlas, 7. Auflage 1994, Lb1, Gl. (2); Grundlagen der Einphasen- und Mehrphasenströmungen, Heinz Brauer, Verlag Sauerländer, Aarau und Frankfurt am Main, 1971). Die kritische Reynolds-Zahl wird mit 2320 angegeben. Unterhalb dieser Zahl liegt ein laminarer Strömungszustand vor.

Da Reaktoreinbauten, wie statische Mischer oder Kolonnenpackungen, die Turbulenz in dem Rohrreaktor erhöhen, ist ihre Verwendung weniger zu empfehlen. Ihr Einsatz ist jedoch nicht ausgeschlossen, solange der Massenstrom der kontinuierlichen Phase bei gegebenem hydraulischen Innendurchmesser so eingestellt wird, dass die kontinuierliche Phase unter laminaren Bedingungen durch den aufrecht stehenden Rohrreaktor strömt. Hingegen können Kühlschlangen oder Kühlfinger zur Abführung der Reaktionswärme in dem Rohrreaktor installiert sein, an denen die kontinuierliche Phase ohne Störung des laminaren Zustands vorbeiströmt.

In einer Ausführungsform des erfindungsgemäßen Verfahrens werden die organische, aldehydhaltige Phase und die wässrige, katalysatorhaltige Phase in den Bodenbereich des Rohrreaktors getrennt aber gleichzeitig eingespeist. Im Bodenbereich können Einbauten vorgesehen werden, welche eine tropfenförmige Verteilung der eintretenden aldehydhaltigen Phase in der wässrigen, katalysatorhaltigen Lösung sicherstellen. Als Einbauten können beispielsweise Düsen, poröse Sinterplatten oder Lanzen installiert sein, durch die die aldehydhaltige Phase in den mit der wässrigen, katalysatorhaltigen Phase gefluteten Rohrreaktor eintritt. In dem Reaktorbereich unterhalb dieser Einbauten befindet sich noch ein Restvolumen, in das die wässrige, katalysatorhaltige Phase einströmt. In dem Reaktorbereich oberhalb der Einbauten zur Zuführung der aldehydhaltigen Phase sind vorzugsweise keine weiteren Mischelemente installiert. Kühleinrichtungen zur Wärmeabfuhr können jedoch vorgesehen werden, ohne dass sie das Strömungsverhalten der kontinuierlichen Phase stören.

Obwohl es am Boden des Rohrreaktors in dem Einströmbereich der beiden Flüssigkeitsströme zu turbulenten Zonen kommen kann, geht das Fließverhalten der kontinuierlichen Phase nach dem Bodenbereich über die Länge des Rohrreaktors in einen laminaren Zustand über. Der aufrecht stehende Rohrreaktor ist mit der kontinuierlichen Phase geflutet, in der die aldehydhaltige, organischen Phase nach ihrem Eintritt in den Bodenbereich tropfenförmig dispergiert ist. Die organischen, aldehydhaltigen Tropfen strömen infolge ihrer geringeren Dichte von der Eintrittsstelle im Bodenbereich des Rohrreaktors her in Richtung Reaktorkopf.

Aufgrund der Dichtedifferenz ist die Aufstiegsgeschwindigkeit der dispergierten organischen Tropfen im Rohrreaktor größer als die der kontinuierlichen wässrigen Phase.

Das Bewegungsverhalten dieser dispergierten Tropfen in der kontinuierlichen Phase kann aus allgemein bekannten strömungstechnischen Zusammenhängen abgeleitet werden (VDI-Wärmeatlas, 10. Auflage 2006, Lda 8, 9 und 14). Zur Beschreibung der Auftriebskräfte hat sich die sogenannte Archimedes-Zahl als zweckmäßig erwiesen:

$$A_r = \frac{d^3_p \cdot g \cdot \rho_K \cdot \Delta\rho}{\eta^2_K} \qquad (2)$$

Darin bedeuteten dp [m] der Durchmesser des Tropfens der dispergierten organischen Phase, g [m/s²] die Fallbeschleunigung, $\rho_K$ [kg/m³] die Dichte der kontinuierlichen Phase, $\Delta\rho$ [kg/m³] die Dichtedifferenz zwischen der kontinuierlichen und dispergierten Phase und $\eta_K$ [Pa·s] die dynamische Viskosität der kontinuierlichen Phase.

Aus der Archimedes-Zahl kann auf die Relativgeschwindigkeit geschlossen werden, wobei bei einer kleinen Archimedes-Zahl der Tropfenaufstieg und damit die Relativgeschwindigkeit von der dispersen Phase in der kontinuierlichen Phase gering ist.

Für den Bereich sehr kleiner Tropfen kann von Tropfen mit einer starren Phasengrenze ausgegangen werden, so dass die Tropfen stabil stetig durch die kontinuierliche Phase hindurch in Richtung Reaktorkopf steigen. Dieser Bereich der starren Phasengrenze wird zahlenmäßig nach:

$$A_r \leq 1{,}83 \cdot K_{F,\Delta\rho}^{0{,}275} \qquad (3)$$

beschrieben, wobei $K_{F,\Delta\rho}$ die modifizierte Flüssigkeitskennzahl bedeutet mit

$$K_{F,\Delta\rho} = \frac{\rho_K \cdot \sigma^3}{g \cdot \eta_K} \cdot \frac{\rho_K}{\Delta\rho} \qquad\qquad (4)$$

in der $\sigma$ [N/m] für die Oberflächenspannung steht und die übrigen Formelzeichen die in Gleichung (2) genannte Bedeutung besitzen.

Nimmt die Tropfengröße und damit die Tropfengeschwindigkeit zu, dann wird die Archimedes-Zahl größer als der gemäß Gleichung (3) errechnete Grenzwert und es kommt zunächst zu einer Bewegung der Phasengrenzfläche des Tropfens, die eine innere Zirkulation im Tropfen bewirkt. Mit weiter zunehmendem Tropfendurchmesser kommt es zu einer Oszillation unter Deformierung des Tropfens bis hin zu einem Zerfall in kleinere Fragmente. Die Stabilität des Tropfens der dispergierten Phase kann gemäß Gleichung (5) abgeschätzt werden.

$$\frac{We_D}{Fr} \cdot \frac{\Delta\rho}{\rho_D} > 9 \qquad\qquad (5)$$

Darin bedeutet $We_D$ die Weber-Zahl der dispergierten Phase und Fr die Froude-Zahl. Diese dimensionslosen Kennzahlen lassen sich nach VDI-Wärmeatlas, 10. Auflage 2006, Lda 14 aus den Stoffdaten berechnen. $\rho_D$[kg/m$^3$] bedeutet die Dichte der dispergierten Phase und $\Delta\rho$ [kg/m$^3$] die Dichtedifferenz zwischen der kontinuierlichen Phase und der dispergierten Phase. Beim Überschreiten des in Gleichung (5) angegebenen Grenzwertes wird die Wahrscheinlichkeit für die ausreichende Stabilität des Tropfens sehr gering.

Für den Betrieb des Rohrreaktors nach dem erfindungsgemäßen Verfahren sind die Bedingungen gemäß den Gleichungen (3) und (5), insbesondere die Tropfengröße der dispergierten Phase, so einzustellen, dass man von stabilen Tropfen mit einer starren Phasegrenze ausgehen kann und die Relativgeschwindigkeit der dispergierten, organischen Phase gegenüber der kontinuierlichen Phase vergleichsweise gering ist. Je kleiner der Tropfen-Durchmesser ist, desto größer ist die Austauschfläche.

In einer weiteren Ausgestaltung des erfindungsgemäßen Verfahrens kann die angesetzte Aldehydmischung und die wässrige, katalysatorhaltige Phase in einem dem Rohrreaktor vorgeschalteten statischen Mischelement dispergiert werden und anschließend auf den Boden des mit der kontinuierlichen Phase gefluteten Rohrreaktors gegeben werden. Derartige statische Mischer werden beispielsweise als Sulzer- oder Kenicks-Mischer kommerziell angeboten mit speziellen Produktlinien für die Dispergierung niedrigviskoser Flüssigkeiten. Bei dieser Ausführungsform des erfindungsgemäßen Verfahrens wird das dispergierte Flüssigkeitsgemisch außerhalb des Rohrreaktors erzeugt und dann in den Rohrreaktor eingeleitet.

Die Verweilzeit in dem vorgeschalteten Mischelement ist nur kurz, so dass die organische Aldehydmischung in der wässrigen Katalysatorphase zwar tropfenförmig dispergiert wird, die Aldoladditions- und kondensationsreaktion aber nur in einem geringen Ausmaß einsetzt. Erst nach Eintritt des mehrphasigen Flüssigkeitsgemisches in den aufrecht stehenden Rohrreaktor setzt die Umsetzung zu den $\alpha,\beta$-ungesättigten Aldehyden in einem nennenswerten Ausmaß ein.

Zusätzliche Einbauten am Reaktorboden sind nicht unbedingt erforderlich jedoch auch nicht ausgeschlossen, solange ein laminarer Strömungszustand der kontinuierlichen Phase über die Reaktorlänge sichergestellt ist.

Bei dem Einsatz eines vorgeschalteten statischen Mischelements kann der Tropfendurchmesser der dispergierten Flüssigkeitstropfen entweder mittels der Weber- und Reynolds-Zahl nach Streiff F.; Recent Prog. Genie Proc. 11. No. 51 (1997) Seite 307 experimentell bestimmt werden. Im Allgemeinen wird der Tropfendurchmesser für statische Mischer auch von dem Hersteller angegeben.

Am Reaktorkopf wird das mehrphasige Flüssigkeitsgemisch abgezogen und in einen Phasentrenner oder Absetzbehälter geleitet, in dem sich die schwerere wässrige Lösung der basisch reagierenden Verbindung separiert. Durch das in der Aldolkondensation gebildete Reaktionswasser wird diese wässrige Lösung verdünnt. Ebenfalls sind wasserlösliche organische Verbindungen, wie durch Aldehydoxidation gebildete Carboxylate, sowie organische Bestandteile aufgrund einer nicht vollständigen Phasentrennung zugegen. Ein Teil der belasteten wässrigen Phase wird daher ausgeschleust.

Die nicht ausgeschleuste wässrige Phase wird als Katalysatorkreislauf zurückgeführt, mit Frischlauge, die die basisch reagierende Verbindung als Aldolisierungskatalysator enthält, ergänzt und auf den Boden des aufrechtstehenden Rohrreaktors gegeben.

Die abgetrennte organische Phase, die Ausgangsaldehyde, das gemischte $\alpha,\beta$- ungesättigte Aldolkondensationsprodukt sowie die durch Selbstaddition einer Aldehydsorte gebildeten $\alpha,\beta$- ungesättigten Aldolkondensationsprodukte und höher siedende Nebenprodukte enthält, kann entweder in nachfolgenden Reinigungsschritten aufgetrennt und anschließend weiterverarbeitet werden, beispielsweise über die vollständige Hydrierung in Alkohole oder über die partielle Hydrierung zum gesättigten Aldehyd, der anschließend zu der entsprechenden Carbonsäure oxidiert werden kann. Ebenfalls ist es möglich, einen Teilstrom der organischen Phase abzuführen und als Flüssigkeitskreislauf wieder in den Rohrreaktor zurückzufahren, um eine vollständige Umsetzung der Ausgangsaldehyde zu erzielen, falls beim geraden Durchgang der Gehalt an den Ausgangsaldehyden in der organischen Phase des Reaktoraustrags noch zu hoch ist.

Die wässrige Lösung enthält eine basisch reagierende Verbindung als Katalysator für die Aldolkondensationsreaktion. Geeignete basische Verbindungen sind Hydroxide, Carbonate, Hydrogencarbonate oder Carboxylate der Alkali- und Erdalkalimetalle, auch in Form ihrer Gemische. Bevorzugt verwendet man Alkalihydroxide wie Natriumhydroxid. Die Konzentration der basisch reagierenden Verbindung beträgt im Allgemeinen 0,1 bis 15 Gew.-%, vorzugsweise 0,1 bis 5 Gew.-%, bezogen auf die wässrige Lösung. Die wässrige Katalysatorlösung wird in einer solchen Menge eingesetzt, dass auf 100 Gewichtsteilen Aldehydmischung von 10 bis 140, vorzugsweise von 20 bis 60 Gewichtsteile basisch reagierende Verbindung, berechnet als Reinsubstanz, verwendet werden.

Für die gemischte Aldolkondensationsreaktion zu $\alpha,\beta$-ungesättigten Aldehyden verwendet man eine Mischung aus zwei aliphatischen Aldehyden unterschiedlicher Kohlenstoffzahl mit 2 bis 5 Kohlenstoffatomen im Molekül, wobei mindestens eine Aldehydsorte zwei Wasserstoffatome an dem zur Carbonylfunktion benachbarten $\alpha$-Kohlenstoffatom tragen muss, damit es nach dem Additionsschritt zur Wasserabspaltung kommt. Beispiele für aliphatische Aldehyde mit zwei Wasserstoffatomen in der $\alpha$-Position sind Acetaldehyd, Propionaldehyd, n-Butyraldehyd, n-Pentanal oder 3-Methylbutanal. Beispiele für aliphatische Aldehyde mit einem Wasserstoffatom an dem $\alpha$-Kohlenstoffatom sind Isobutyraldehyd oder 2-Methylbutanal.

Die beiden aliphatischen Aldehyde mit der unterschiedlichen Kohlenstoffzahl werden in einem Molverhältnis von 1:1 bis 5:1, vorzugsweise von 1:1 bis 3:1, abgemischt und nach dem erfindungsgemäßen Verfahren zu den gemischten $\alpha,\beta$-ungesättigten Aldolkondensationsprodukt umgesetzt. Das erfindungsgemäße Verfahren ist besonders für die Mischaldolisierung von Acetaldehyd mit n-Butyraldehyd zum 2-Ethylbutenal, von Acetaldehyd mit Propionaldehyd zum 2-Methylbutenal, von Acetaldehyd mit n-Pentanal zum 2-Ethylpentenal, von Propionaldehyd mit n-Butyraldehyd zum 2-Ethylpentenal und 2-Methylhexenal, von Propionaldehyd mit n-Pentanal zum 2-Methylheptenal und 2-Propylpentenal oder von n-Butyraldehyd mit n-Pentanal zum 2-Ethylheptenal und 2-Propylhexenal geeignet. Die Einsatzaldehyde können neben der jeweiligen Hauptkomponente auch noch geringe Mengen an herstellungsbedingten Verunreinigungen enthalten. Beispielsweise kann n-Butyraldehyd noch geringe Mengen an iso-Butyraldehyd enthalten, falls n-Butyraldehyd über die Propylenhydroformylierung hergestellt wird. In gleicher Weise kann das über Buten-1-Hydroformylierung zugängliche n-Pentanal noch geringe Mengen an 2-Methylbutanal aufweisen.

Die Bildung der $\alpha,\beta$- ungesättigten Aldolkondensationsprodukte aus der Selbstkondensation einer Aldehydsorte, beispielsweise die Bildung von Crotonaldehyd und 2-Ethylhexenal bei der Mischaldolisierung von Acetaldehyd mit n-Butyraldehyd, mindert zwar die Ausbeute an dem gemischten $\alpha,\beta$- ungesättigten Aldolkondensationsprodukt, doch diese Produkte können beispielsweise nach der Kompletthydrierung als Alkohole vermarktet werden. Um jedoch den Anfall an hochsiedenden Nebenprodukten, die keine Wertprodukte darstellen, zu reduzieren, wird die Aldolkondensationsreaktion bei Temperaturen bis 90°C, vorzugsweise im Bereich von 10°C bis 65°C und insbesondere im Bereich von 30°C bis 60°C durchgeführt. Die Reaktion wird unter Eigendruck oder leichtem Überdruck betrieben, obwohl die Anwendung höherer Drücke, beispielsweise bis 0,8 MPa, nicht ausgeschlossen ist.

Bei der kontinuierlichen Fahrweise wird der Rohrreaktor bei einer Reaktorbelastung V/Vh mit dem Aldehydgemisch pro Reaktorvolumen und Zeit von 0,1 bis 1,3, vorzugsweise von 0,8 bis 1,3, betrieben. Eine höhere Reaktorbelastung mit dem Aldehydgemisch ist zu vermeiden, weil dann die Aldolkondensationsreaktion nicht mehr vollständig erfolgt und die abgeführte organische Phase einen zu hohen Restgehalt an den Ausgangsaldehyden aufweist. Bei zu geringer Reaktorbelastung wird die Anlagenkapazität nicht optimal ausgenutzt. Beim Anfahren des kontinuierlich durchgeführten Verfahrens wird der aufrecht stehende Rohrreaktor zunächst mit der wässrigen Lösung der basisch reagierenden Verbindung geflutet. Anschließend werden der organische, aldehydhaltige Flüssigkeitsstrom und die wässrige Lösung der basisch reagierenden Verbindung über den Boden des Rohrreaktors zugeführt. Dabei können der organische und wässrige Stoffstrom getrennt aber gleichzeitig über den Boden des Rohrreaktors zugeführt werden, so dass die Bildung des organisch/wässrigen Mehrphasensystems innerhalb des Rohrreaktors erfolgt. Ebenfalls können der organische und der wässrige Stoffstrom in einem dem Rohrreaktor vorgeschalteten statischen Mischelement vermischt werden, so dass das organisch/wässrige Mehrphasensystem am Boden in den Rohrreaktor eingeleitet wird.

Durch den Betrieb des Rohrreaktors im Gleichstrom mit der kontinuierlichen Phase unter laminaren Bedingungen kann, bezogen auf den Massenstrom der wässrigen Phase, ein vergleichsweise hoher Massenstrom an der organischen Phase durch den Reaktor geführt werden. Im Allgemeinen liegt das Verhältnis der Massenströme von der Aldehydmischung zu der wässrigen Lösung der basisch reagierenden Verbindung im Bereich 1 zu (5-56), vorzugsweise 1 zu (10-32). Durch den vergleichsweise hohen Anteil an organischem Produkt im Rohrreaktor wird die Anlageneffizienz deutlich verbessert. Auch ermöglicht der Betrieb des Rohrreaktors eine einfache, energieschonende Reaktionsführung ohne die Verwendung aufwendiger Mischpumpen und wartungsintensiver mechanischer Anlagenteile sowie aufwendiger Säuleneinbauten, wie beispielsweise in den Rohrreaktor eingebaute statische Mischer.

Auch erlaubt diese Reaktionsführung infolge der Verdünnung der eingesetzten Aldehyde mit der wässrigen Lösung der basisch reagierenden Verbindung eine gleichmäßige Wärmeabfuhr und vermeidet die Bildung von Temperaturspitzen mit unkontrollierten Überreaktionen, so dass die gemischte Aldolkondensationsreaktion der aliphatischen Aldehyde sicherheitstechnisch sehr gut kontrolliert werden kann und sehr selektiv zu den gewünschten gemischten α,β-ungesättigten Aldehyden verläuft. Die Bildung von hochsiedenden Nebenprodukten kann daher zurückgedrängt werden.

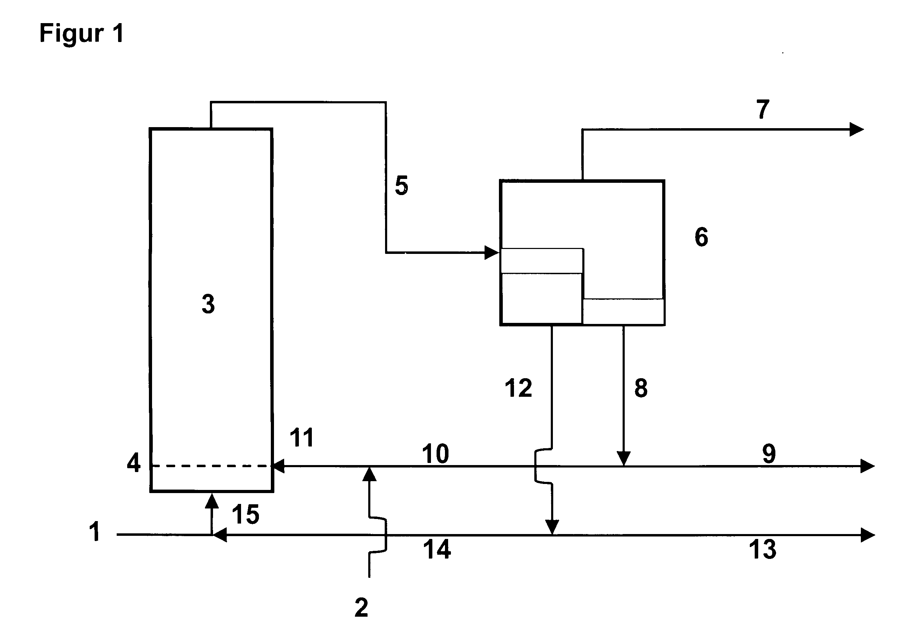

Das erfindungsgemäße Verfahren wird im Folgenden anhand des Prinzipschemas gemäß Figur 1 näher erläutert. Das erfindungsgemäße Verfahren ist aber nicht auf die in der Zeichnung dargestellte Ausführungsform beschränkt. Das separate Abmischen der beiden aliphatischen Aldehyde ist nicht dargestellt.

Über die Leitung (1) wird eine frische wässrige Lösung einer basisch reagierenden Verbindung und über die Leitung (2) frisches Aldehydgemisch herangeführt und nach dem Vermischen mit den jeweiligen Kreislaufströmen an dem Boden des Rohrreaktors (3) eingeleitet, in dem am Boden Vorrichtungen (4) installiert sind, durch die die einströmende organische Lösung in Flüssigkeitstropfen zerteilt wird, die in der wässrigen Phase verteilt in Richtung Reaktorkopf strömen. Zur Wärmeabfuhr können in dem Rohrreaktor (3) Kühlschlangen oder Kühlfinger installiert sein, die jedoch das laminare Strömungsverhalten der kontinuierlichen Phase nicht stören (nicht in Figur 1 eingezeichnet). Am Reaktorkopf wird über die Leitung (5) der flüssige Reaktoraustrag abgeführt und in einen Absetzbehälter (6) geleitet, in dem sich die leichtere organische Phase von der schwereren wässrigen Phase trennt. Gasförmige Anteile werden über die Leitung (7) abgeführt. Die abgesetzte organische Phase, die das gemischte α,β- ungesättigte Aldolkondensationsprodukt, unumgesetzte Aldehyde und α,β- ungesättigte Aldolkondensationsprodukte aus der Selbstaddition enthält, verlässt den Absetzbehälter (6) über die Leitung (8). Ein Teilstrom wird über die Leitung (9) ausgeschleust, der in nachfolgenden Destillationseinrichtungen in die verschiedenen organischen Bestandteile aufgetrennt wird (nicht in Figur 1 gezeigt). Der andere Teilstrom wird als Kreislauf über die Leitung (10) zurückgeführt und mit über Leitung (2) zugeführtem frischen Aldehydgemisch vereinigt und über die Leitung (11) am Boden in den Reaktor (3) eingespeist. Die im Absetzbehälter (6) anfallende wässrige Lösung der basisch reagierende Verbindung fliest über Leitung (12) ab und wird teilweise über die Leitung (13) entfernt. Der andere Teilstrom fliest als Katalysatorkreislauf über die Leitung (14) zurück, wird mit über Leitung (1) zugeführter frischer wässriger Lösung der basisch reagierenden Verbindung vereinigt und über die Leitung (15) am Boden in den Reaktor (3) gepumpt.

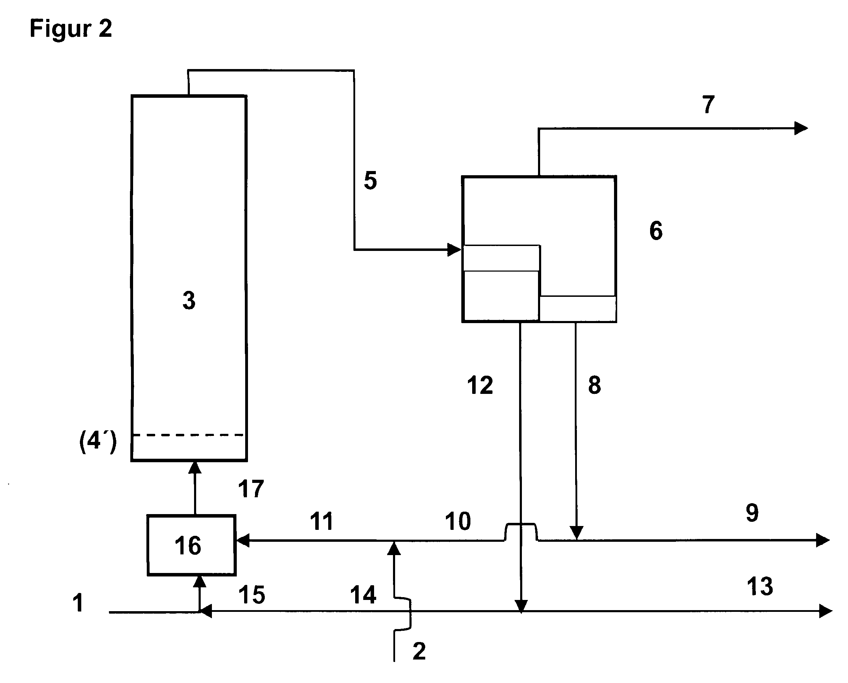

In der nachfolgenden Figur 2 ist eine weitere Ausführung des erfindungsgemäßen Verfahrens unter Verwendung eines dem Rohrreaktor vorgeschalteten statischen Mischers dargestellt. Die über Leitung (15) herangeführte wässrige Lösung und die über Leitung (11) herangeführte organische Lösung werden in dem statischen Mischer (16) dispergiert. Das flüssige Mehrphasensystem tritt dann über die Leitung (17) auf den Boden des Rohrreaktors (3). Auf die schematisch angedeuteten Einbauten (4') kann bei dieser Ausführungsform verzichtet werden, obwohl Vorrichtungen im Bodenbereich des Rohrreaktors nicht ausgeschlossen sind, solange ein laminares Strömungsverhalten der kontinuierlichen Phase über die Länge des Rohrreaktors gewährleistet ist.

Im Folgenden wird das erfindungsgemäße Verfahren anhand einiger Beispiele näher erläutert, es ist jedoch nicht auf die beschriebenen Ausführungsformen beschränkt.

Versuchsaufbau

Ein Rohrreaktor mit einem Volumen von 0,19 Liter wurde zunächst mit einer wässrigen Natronlauge mit einer Konzentration von 2,5 Gew.-% geflutet. Über den Reaktorboden führte man anschließend kontinuierlich das Gemisch zweier aliphatischer Aldehyde mit unterschiedlicher Kohlenstoffzahl sowie die 2,5 Gew.-%-ige wässrige Natronlauge in den Rohrreaktor. Wurde ohne statischen Mischer gearbeitet, leitete man beide Stoffströme getrennt aber gleichzeitig auf den Boden des Rohrreaktors. Bei den Versuchen mit einem statischen Mischer wurde ein Sulzer Mischer vom Typ SMX DN4 verwendet. Das Mischelement war vor dem Reaktorboden außerhalb des Rohrreaktors angebracht. Darin wurden die organische und wässrige Lösung miteinander vermischt und anschließend wurde das flüssige Mehrphasensystem mit der dispergierten organischen Phase auf den Rohrreaktor gegeben. Bei beiden Ausführungsformen durchströmte die dispergierte organische Phase tropfenförmig die kontinuierliche wässrige Phase.

Am Reaktorkopf entnahm man das mehrphasige Reaktionsgemisch und führte es in einen Absetzbehälter. Aus den abgeschiedenen flüssigen Phasen wurde sowohl ein wässriger als auch ein organischer Kreislaufstrom wieder in den Rohrreaktor zurückgeführt. Die nicht zurückgeführte wässrige Phase wurde ausgeschleust während die nicht zurückgeführte organische Phase auf ihren Wertproduktgehalt hin gaschromatographisch analysiert wurde.

Die Reaktionsbedingungen, die kontinuierliche Zufuhr der Einsatzstoffe sowie die Kreislaufströme wurden gemäß den Bedingungen der nachfolgenden Tabelle 1 eingestellt. In der Tabelle 1 ist ebenfalls die gaschromatographisch ermittelte Zusammensetzung des organischen Produkts, wasserfrei, angegeben (in %). Die Versuche wurden bei einem leichten Überdruck von 0,02 MPa betrieben.

7

Beispiele 1-4:

Tabelle 1: Herstellung von 2-Ethylbutenal durch Mischaldolisierung von Acetaldehyd mit n-Butyraldehyd

| Beispiele | 1 | 2 | 3 | 4 |
|---|---|---|---|---|
| Temperatur [°C] | 40 | 40 | 40 | 57 |
| Acetaldehyde [g/h] | 25 | 25 | 25 | 25 |
| n-Butanal [g/h] | 100 | 100 | 100 | 100 |
| Katalysator-Kreislauf [kg/h], wässrige NaOH | 1 | 1 | 4 | 1 |
| | | | | |
| **Lauge** | | | | |
| Frisch NaOH (2,5-Gew.-%) [mL/h] | 18 | 18 | 15 | 15 |
| n-Butanal/Acetaldehyd (molar) | 2,5 | 2,5 | 2,5 | 2,5 |
| Sulzermischer | ohne | mit | mit | mit |
| | | | | |
| **Analyse [GC%]** | | | | |
| Vorlauf | 0,1 | 0,1 | 0,1 | 0,1 |
| *n*-Butanal | 14,5 | 9,3 | 5,6 | 4,4 |
| Crotonaldehyd + Zwischenlauf | 0,3 | 0,4 | 0,4 | 0,2 |
| 2-Ethylbutenal | 20,4 | 26,3 | 26,7 | 25,3 |
| Zwischenlauf | 2,6 | 3,4 | 3,0 | 2,7 |
| 2-Ethyl-2-hexenal | 32,5 | 35,2 | 35,6 | 39,2 |
| Nachläufe | 29,6 | 25,3 | 28,6 | 28,1 |
| | | | | |
| Ausbeute an 2-Ethylbutenal, bezogen auf Acetaldehydeinsatz | 41 | 55 | 53 | 49 |

Für das Beispiel 3 sind die hydraulischen Daten in Bezug auf die Querschnittsbelastung des Rohrreaktors in Tabelle 2 zusammengefasst. Der Einsatzmassenstrom in den Rohrreaktor beträgt für Beispiel 3 $m_x$ = 4,140 kg/h, der sich aus dem Eintrag an Acetaldehyd und n-Butanal sowie an Katalysator-Kreislauf und Frisch-NaOH zusammensetzt.

Tabelle 2: Hydraulische Kennzahlen bezogen auf die wässrige kontinuierliche Phase; exemplarisch für Beispiel 3

| | |
|---|---|
| Dichte $\rho$ [kg/m$^3$] | 990 |
| Dynamische Viskosität $\eta$ [Pa·s] | 0,000677 |
| Hydraulischer Innendurchmesser des Rohrreaktors [m] | 0,009 |
| Massenstrom der Reaktanden [kg/h] | 4,140 |

Unter Verwendung von Gleichung (1) ergibt sich aus diesen Kennzahlen eine Reynolds-Zahl von 239. Da bei den Beispielen 1, 2 und 4 mit einem geringeren Einsatzmassenstrom gearbeitet wurde, folgt nach Gleichung (1) eine noch kleinere Reynolds-Zahl. Bei den Beispielen 1-4 liegen somit in der kontinuierlichen Phase stabil laminare Strömungsbedingungen vor.

Aufgrund der Dichtedifferenz ist die Aufstiegsgeschwindigkeit der dispergierten Phase in dem Rohrreaktor größer als die der kontinuierlichen Phase. Für das Beispiel 3 ergeben sich folgende hydraulische Kennzahlen für die dispergierten Phase unter Verwendung der im VDI-Wärmeatlas, 10. Auflage 2006, Lda 8, Lda 9 und Lda 14 beschriebenen formelmäßigen Zusammenhänge.

Tabelle 3: Hydraulische Kennzahlen bezogen auf die organische dispergierte Phase; exemplarisch für Beispiel 3

| Dichtedifferenz wässrig-organisch [kg/m³] | 209,00 |
|---|---|
| Tropfendurchmesser * [m] | 0,0008 |
| Relativgeschwindigkeit organisch-wässrig [m/s] | 0,096 |
| Reynolds-Zahl | 106 |
| Archimedes-Zahl | 1908 |
| Weber-Zahl | 0,08 |
| Froude-Zahl | 1,24 |
| Konsistenzfaktor $K_{F, \Delta\rho}$ | $6,46 \cdot 10^{11}$ |
| * Herstellerangaben | |

Unter Verwendung von Gleichung (5) errechnet sich ein Stabilitätswert von 0,02, so dass die während der Strömung angreifenden Kräfte nicht zu einem Zerfall der Tropfen führen. Ebenfalls ist die Bedingung in Gleichung (2) erfüllt, so dass die in der kontinuierlichen Phase aufsteigenden organischen Tropfen als stabile Tropfen mit einer starren Phasengrenze beschrieben werden können. In analoger Weise können die hydraulischen Kennzahlen für die organische, dispergierte Phase für die Beispiele 1, 2 und 4 errechnet und so die Tropfenstabilitäten abgeschätzt werden.

Beispiele 5-7 (Mischaldolisierung von n-Butyraldehyd mit n-Pentanal) sowie Beispiele 8 und 9 (Mischaldolisierung von Acetaldehyd und n-Pentanal)

Für die Durchführung der Beispiele 5-7 sowie 8 und 9 diente der in den Beispielen 1-4 verwendete Versuchsaufbau.

Die Reaktionsbedingungen, die kontinuierliche Zufuhr der Einsatzstoffe sowie die Kreislaufströme wurden gemäß den Bedingungen der nachfolgenden Tabellen 4 und 5 eingestellt. In den Tabelle 4 und 5 sind ebenfalls die gaschromatographisch ermittelte Zusammensetzung des organischen Produkts, wasserfrei, angegeben (in %). In allen Versuchen wurde ein Sulzermischer des Typs SMX DN4 eingesetzt, der vor dem Reaktorboden außerhalb des Rohrreaktors installiert war. Die Versuche wurden bei einem leichten Überdruck von 0,02 MPa durchgeführt.

Beispiele 5-7:

Tabelle 4: Herstellung von 2-Propyl-2-hexenal und 2-Ethyl-2-heptenal durch Mischaldolisierung von n-Butyraldehyd mit n-Pentanal

| Beispiele | 5 | 6 | 7 |
|---|---|---|---|
| Temperatur [°C] | 55 | 80 | 80 |
| n-Butanal [g/h] | 55 | 78 | 84 |
| n-Pentanal [g/h] | 65 | 47 | 41 |
| Katalysator-Kreislauf [kg/h]; wässr. NaOH | 2 | 2 | 2 |
| | | | |
| Lauge | | | |
| Frisch NaOH (2,5-Gew.-%) [mL/h] | 15 | 15 | 15 |
| n-Butanal/n-Pentanal (molar) | 1 | 2 | 2,5 |
| | | | |
| Analyse [GC%] | | | |
| Vorlauf | 0,1 | 0,1 | 0,1 |
| n-Butanal | 3,8 | 1,3 | 1,4 |
| 2-Methylbutanal | 0,1 | 0,1 | 0,1 |

(fortgesetzt)

| Analyse [GC%] | | | |
|---|---|---|---|
| n-Butanol | 0,1 | 0,1 | 0,1 |
| n-Pentanal | 16,9 | 5,2 | 5,6 |
| Zwischenlauf | 0,3 | 0,4 | 0,4 |
| 2-Ethyl-2-hexenal | 21,6 | 40,8 | 47,9 |
| Zwischenlauf | 0,1 | 0,2 | 0,2 |
| 2-Propyl-2-hexenal | 13,5 | 15,7 | 13,9 |
| 2-Ethyl-2-heptenal | 13,0 | 17,0 | 15,1 |
| Zwischenlauf | 5,7 | 3,6 | 3,3 |
| 2-Propyl-2-heptenal | 12,6 | 8,3 | 6,3 |
| Nachläufe + Höhersieder | 12,2 | 7,2 | 5,6 |
| Ausbeute (%) an 2-Propylhexenal und 2-Ethylheptenal bezogen auf n-Pentanaleinsatz | 42 | 86 | 77 |

Beispiele 8 und 9:

Tabelle 5: Herstellung von 2-Ethylpentenal durch Mischaldolisierung von Acetaldehyd mit n-Pentanal

| Beispiele | 8 | 9 |
|---|---|---|
| Temperatur [°C] | 40 | 50 |
| Acetaldehyd [g/h] | 21 | 21 |
| n-Pentanal [g/h] | 102 | 102 |
| Katalysator-Kreislauf [kg/h], wässr. NaOH | 2 | 2 |
| | | |
| **Lauge** | | |
| Frisch NaOH (2,5-Gew.-%) [mL/h] | 20 | 15 |
| n-Pentanal/Acetaldehyd (molar) | 2,5 | 2,5 |
| | | |
| **Analyse [GC%]** | | |
| Vorlauf | 0,1 | 0,1 |
| Acetaldehyd | 0,2 | 0,1 |
| 2-Butenal | 0,1 | 0,1 |
| 2-Methylbutanal | 0,1 | 0,4 |
| n-Pentanal | 26,0 | 20,3 |
| Zwischenlauf | 0,4 | 0,6 |
| 2-Ethylpentenal | 22,4 | 23,5 |
| 2-Heptenal | 1,5 | 1,9 |
| Zwischenlauf | 4,3 | 3,5 |
| 2-Propylheptenal | 15,1 | 23,4 |
| Nachlauf | 29,8 | 26,1 |

(fortgesetzt)

| Analyse [GC%] | | |
|---|---|---|
| Ausbeute an 2-Ethylpentenal, bezogen auf Acetaldehydeinsatz | 54 | 55 |

**Patentansprüche**

1. Kontinuierliches Verfahren zur Durchführung einer mehrphasigen Aldolkondensationsreaktion zu gemischten $\alpha,\beta$-ungesättigten Aldehyden in einem Rohrreaktor durch Umsetzung von zwei aliphatischen Aldehyden mit unterschiedlicher Kohlenstoffzahl, die 2 bis 5 Kohlenstoffatome im Molekül enthalten, **dadurch gekennzeichnet, dass** die aliphatischen Aldehyde in einem Molverhältnis von 1:1 bis 5:1 separat gemischt werden, danach die Aldehydmischung und eine wässrige Lösung einer basisch reagierenden Verbindung über den Boden in einen aufrecht stehenden Rohrreaktor im Gleichstrom eingeleitet werden, wobei die wässrige Lösung der basisch reagierenden Verbindung die kontinuierliche Phase bildet, die unter laminaren Bedingungen durch den Rohrreaktor strömt, und die Aldehydmischung die in der kontinuierlichen Phase tropfenförmig dispergierte Phase bildet und wobei die Aldolkondensationsreaktion bei einer Temperatur bis 90°C durchgeführt wird und wobei der Rohrreaktor mit einer Belastung V/Vh mit dem Aldehydgemisch pro Reaktorvolumen und Zeit von 0,1 bis 1,3 betrieben wird.

2. Verfahren gemäß Anspruch 1, **dadurch gekennzeichnet, dass** die aliphatischen Aldehyde in einem Molverhältnis von 1:1 bis 3:1 separat gemischt werden.

3. Verfahren gemäß Anspruch 1 oder 2, **dadurch gekennzeichnet, dass** die Aldolkondensationsreaktion bei Temperaturen von 10°C bis 65°C und insbesondere von 30°C bis 60°C durchgeführt wird.

4. Verfahren gemäß einem oder mehreren der Ansprüche 1 bis 3, **dadurch gekennzeichnet, dass** auf 100 Gewichtsteilen Aldehydmischung 10 bis 140, vorzugsweise 20 bis 60 Gewichtsteile, basisch reagierende Verbindung, berechnet als Reinsubstanz, verwendet werden.

5. Verfahren gemäß einem oder mehreren der Ansprüche 1 bis 4, **dadurch gekennzeichnet, dass** das Verhältnis der Massenströme von der Aldehydmischung zu der wässrigen Lösung der basisch reagierenden Verbindung im Bereich 1 zu (5-56), vorzugsweise 1 zu (10-32), liegt.

6. Verfahren gemäß einem oder mehreren der Ansprüche 1 bis 5, **dadurch gekennzeichnet, dass** man als aliphatische Aldehyde Acetaldehyd, Propionaldehyd, n-Butyraldehyd, iso-Butyraldehyd, n-Pentanal, 2-Methylbutanal oder 3-Methylbutanal verwendet.

7. Verfahren gemäß einem oder mehreren der Ansprüche 1 bis 6, **dadurch gekennzeichnet, dass** die Aldolkondensationsreaktion mit Acetaldehyd und n-Butyraldehyd, mit Acetaldehyd und n-Pentanal oder mit n-Butyraldehyd und n-Pentanal durchgeführt wird.

8. Verfahren gemäß einem oder mehreren der Ansprüche 1 bis 7, **dadurch gekennzeichnet, dass** die dispergierten Tropfen der Aldehydmischung in der kontinuierlichen Phase der Bedingung $A_r \leq 1,83 \cdot K_{F,\Delta\rho}^{0,275}$ genügen.

**Claims**

1. Continuous process for performing a polyphasic aldol condensation reaction to give mixed $\alpha,\beta$-unsaturated aldehydes in a tubular reactor by reaction of two aliphatic aldehydes which have different carbon numbers and contain 2 to 5 carbon atoms in the molecule, **characterized in that** the aliphatic aldehydes are mixed separately in a molar ratio of 1:1 to 5:1, then the aldehyde mixture and an aqueous solution of a basic compound are introduced via the base into an upright tubular reactor in cocurrent, the aqueous solution of the basic compound forming the continuous phase which flows through the tubular reactor under laminar conditions, and the aldehyde mixture forming the phase dispersed in droplet form in the continuous phase and the aldol condensation reaction being performed at a temperature up to 90°C and the tubular reactor being operated with a space velocity V/Vh of the aldehyde mixture per unit reactor volume and time of 0.1 to 1.3.

**2.** Process according to Claim 1, **characterized in that** the aliphatic aldehydes are mixed separately in a molar ratio of 1:1 to 3:1.

**3.** Process according to Claim 1 or 2, **characterized in that** the aldol condensation reaction is performed at temperatures of 10°C to 65°C and especially of 30°C to 60°C.

**4.** Process according to one or more of Claims 1 to 3, **characterized in that** 10 to 140 and preferably 20 to 60 parts by weight of basic compound are used per 100 parts by weight of aldehyde mixture, calculated as the pure substance.

**5.** Process according to one or more of Claims 1 to 4, **characterized in that** the ratio of the mass flow rate of the aldehyde mixture to that of the aqueous solution of the basic compound is in the range of 1 to (5-56), preferably 1 to (10-32).

**6.** Process according to one or more of Claims 1 to 5, **characterized in that** the aliphatic aldehydes used are acetaldehyde, propionaldehyde, n-butyraldehyde, isobutyraldehyde, n-pentanal, 2-methylbutanal or 3-methylbutanal.

**7.** Process according to one or more of Claims 1 to 6, **characterized in that** the aldol condensation reaction is performed with acetaldehyde and n-butyraldehyde, with acetaldehyde and n-pentanal, or with n-butyraldehyde and n-pentanal.

**8.** Process according to one or more of Claims 1 to 7, **characterized in that** the dispersed droplets of the aldehyde mixture in the continuous phase satisfy the condition $A_r \leq 1.83 \cdot K_{F, \Delta\rho}^{0.275}$.

**Revendications**

**1.** Procédé continu pour la réalisation d'une réaction de condensation aldol à plusieurs phases en aldéhydes mixtes $\alpha,\beta$-insaturés dans un réacteur tubulaire par transformation de deux aldéhydes aliphatiques présentant un indice de carbone différent, qui contiennent 2 à 5 atomes de carbone dans la molécule, **caractérisé en ce que** les aldéhydes aliphatiques sont mélangés séparément dans un rapport molaire de 1:1 à 5:1, puis on introduit le mélange d'aldéhydes et une solution aqueuse d'un composé à réaction basique via le fond dans un réacteur tubulaire vertical en flux parallèles, la solution aqueuse du composé à réaction basique formant la phase continue, qui s'écoule dans des conditions laminaires au travers du réacteur tubulaire, et le mélange d'aldéhydes formant la phase dispersée sous forme de gouttes dans la phase continue et la réaction de condensation aldol étant réalisée à une température jusqu'à 90°C et le réacteur tubulaire étant exploité à une charge V/Vh du mélange d'aldéhydes par volume de réacteur et par temps de 0,1 à 1,3.

**2.** Procédé selon la revendication 1, **caractérisé en ce que** les aldéhydes aliphatiques sont mélangés séparément dans un rapport molaire de 1:1 à 3:1.

**3.** Procédé selon la revendication 1 ou 2, **caractérisé en ce que** la réaction de condensation aldol est réalisée à des températures de 10°C à 65°C et en particulier de 30°C à 60°C.

**4.** Procédé selon l'une ou plusieurs des revendications 1 à 3, **caractérisé en ce qu'**on utilise par 100 parties en poids de mélange d'aldéhydes, 10 à 140, de préférence 20 à 60 parties en poids de composé à réaction basique, calculées sous forme de substance pure.

**5.** Procédé selon l'une ou plusieurs des revendications 1 à 4, **caractérisé en ce que** le rapport des flux massiques du mélange d'aldéhydes à la solution aqueuse du composé à réaction basique se situe dans la plage de 1:(5-56), de préférence de 1:(10-32).

**6.** Procédé selon l'une ou plusieurs des revendications 1 à 5, **caractérisé en ce qu'**on utilise, comme aldéhydes aliphatiques, de l'acétaldéhyde, du propionaldéhyde, du n-butyraldéhyde, de l'iso-butyraldéhyde, du n-pentanal, du 2-méthylbutanal ou du 3-méthylbutanal.

**7.** Procédé selon l'une ou plusieurs des revendications 1 à 6, **caractérisé en ce que** la réaction de condensation aldol est réalisée avec de l'acétaldéhyde et du n-butyraldéhyde, avec de l'acétaldéhyde et du n-pentanal ou avec du n-butyraldéhyde et du n-pentanal.

**8.** Procédé selon l'une ou plusieurs des revendications 1 à 7, **caractérisé en ce que** les gouttes dispersées du mélange d'aldéhydes dans la phase continue satisfont à la condition $A_r \leq 1,83 * K_{F,\Delta\rho}^{0.275}$.

**Figur 1**

EP 2 681 181 B1

**Figur 2**

EP 2 681 181 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9507254 A1
- EP 0420035 A1
- US 3763247 A
- DE 927626
- US 2468710 A
- GB 761203 A
- EP 1106596 A2
- WO 2010105892 A1

**In der Beschreibung aufgeführte Nicht-Patentliteratur**

- **G. DÜMBGEN ; D. NEUBAUER.** *Chemie -Ing.-Tech.,* 1969, vol. 41, 974